# EUROPEAN PATENT APPLICATION

(11) **EP 3 981 330 A2**
(43) Date of publication of application: **13.04.2022**
(21) Application number: 21201634.9
(22) Date of filing: 08.10.2021
(51) Int. Cl.: A61B 5/1486, A61B 5/1477, G01N 27/327

(54) **ELECTROCHEMICAL PHYSIOLOGICAL SENSOR**

(30) Priority: 12.10.2020 IT 202000023914; 12.10.2020 IT 202000023917; 12.10.2020 IT 202000023923
(71) Applicant: Biometrica S.r.l., 43040 Varano d'Melgari Parma (IT)
(72) Inventor: Beccatelli, Matteo, 43041 Bedonia (IT); Beccatelli, Tommaso, 43041 Bedonia (IT); Bercella, Franco, 43040 Varano De Melegari (IT)
(74) Representative: Lunati & Mazzoni S.r.L.

(57) **Abstract**

It is provided an electrochemical physiological sensor (1) configured to come into contact with the body fluid and comprising an absorber (2) of a body fluid defining a first surface (2a) configured to at least come into contact with said body fluid and a second surface (2b) opposite to the first surface (2a); and a circuit (3) deposited on the second surface (2b) and configured to contact the body fluid when absorbed by the absorber (2); the absorber (2) has a porosity substantially between 40% and 70% so that the body fluid is absorbed by the absorber (2) and the circuit (3) is deposited without penetrating the absorber (2).

## Description

The present invention relates to an electrochemical physiological sensor of the type specified in the preamble of the first claim.

In particular, the invention relates to a sensor for detecting a physiological condition of a user, preferably human, and more precisely an electrochemical physiological sensor.

As known, physiological sensors are tools for detecting a physiological parameter and therefore monitoring the physiological state of the wearer. A particularly common example of a physiological sensor is the heart rate monitor which, thanks to electrodes in contact with the skin, monitors the electrical voltage of the heart and therefore detects its beating frequency.

Another example of physiological sensors is to be identified in electrochemical physiological sensors, that is, in those sensors that detect a physiological parameter through a body fluid in a non-invasive way.

These physiological sensors are mostly equipped with an operating control board of the circuit and a detection system such as potentiometric, amperometric type and, in recent years, an organic electrochemical transistor (OECT), or rather a device belonging to the class of transistors with electrolytic gate (EGTs) which is characterized by the fact that the electrolyte (in this case identified by the body fluid) interfaces, on the one hand, with a gate and on the other it interacts with a channel. The OECT is typically made by depositing on a substrate an electrically conductive polymer (polypyrrole, polyaniline, PEDOT-PSS) so as to obtain a circuit placed in connection with the control board and having three electrodes called source, drain and gate.

Source and drain are connected to each other through a conductive polymer identifying the transistor channel available in contact with the electrolyte.

The gate is used to control the doping level and therefore the conductivity of the polymer.

The operation of an electrochemical physiological sensor can be described as follows. The drain is connected to ground and a potential is applied to the source so as to make a current flow in the channel between the source and drain measured as a function of the potential applied to the gate. The control board commands the application of a potential, for example positive, to the gate causing the introduction of positive ions from the fluid to the electrically conductive polymer which, in turn, causes a variation of the aforesaid current. This current variation is used by the control board to determine the ion concentration in the electrolyte.

Known electrochemical physiological sensors are described in US2020138343A1, US2019131555A1 and EP1746413A2.

The known art described includes some important drawbacks.

A first drawback is represented by the low quality of these electrochemical physiological sensors and therefore by their inability to perform an incorrect analysis of the electrolyte.

In particular, the deposition of the conductive polymer on the fabric is relatively imprecise and therefore unable to precisely define the desired shape of the electrodes with a current flow different from that expected. This aspect therefore negatively affects the measurement of the current variation and therefore the measurement of the ion concentration in the electrolyte.

This incorrect analysis of the electrolyte is also due to the fact that the known electrochemical physiological sensors, being the present technology relatively new, do not have software capable of carrying out a correct evaluation of the data collected.

It is also pointed out that the above-described inability to perform a correct analysis is due to the fact that the peculiar structure of the OECT does not allow to have an optimal connection between the circuit and the control board.

This aspect is accentuated by the difficulty of having a circuit correctly in contact with the electrolyte.

Finally, it is pointed out that currently known electrochemical physiological sensors have particularly high production costs and times and at the same time are not characterized by high reliability and duration.

In this situation, the technical task at the base surface of the present invention is to devise an electrochemical physiological sensor capable of substantially obviating at least part of the aforementioned drawbacks.

Within the scope of said technical task, an important object of the invention is to obtain an electrochemical physiological sensor of high constructive quality and therefore capable of carrying out a precise analysis of the electrolyte.

Another important object of the invention is to provide an electrochemical physiological sensor of relatively reduced production costs and times.

A not secondary object of the invention is to have an electrochemical physiological sensor characterized by improved reliability and duration.

The technical task and the specified aims are achieved by an electrochemical physiological sensor as claimed in the annexed claim 1. Examples of preferred embodiment are described in the dependent claims.

The characteristics and advantages of the invention are clarified below by the detailed description of preferred embodiments of the invention, with reference to the accompanying figures, in which:
the **Fig. 1** shows, in scale, a physiological electrochemical sensor according to the invention;
the **Fig. 2** illustrates, in scale, an assembly of the physiological electrochemical sensor according to the invention; and
the **Fig. 3** shows, in scale, an exploded view of Fig. 2.

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

The measurements and data reported in this text are to be considered, unless otherwise indicated, as performed in the International Standard Atmosphere ICAO (ISO 2533:1975).

Unless otherwise specified, as results in the following discussions, terms such as "treatment", "computing", "determination", "calculation", or similar, refer to the action and/or processes of a computer or similar electronic calculation device that manipulates and/or transforms data represented as physical, such as electronic quantities of registers of a computer system and/or memories in, other data similarly represented as physical quantities within computer systems, registers or other storage, transmission or information displaying devices.

With reference to the Figures, the electrochemical physiological sensor according to the invention is globally indicated with the number **1**.

It can be used for the analysis of one or more fluids and in detail body fluids of an animal or preferably human user. In particular, the electrochemical physiological sensor 1 is configured to absorb and then analyse one or more body fluids (for example sweat) taken by a user and/or leaving the skin.

The electrochemical physiological sensor 1 can be wearable. It can thus be integrated into a garment preferably in a solvable manner so that it can be replaced. Suitably, a garment can comprise at least one electrochemical physiological sensor 1 configured to contact the skin when the garment is worn.

The electrochemical physiological sensor 1 can comprise an absorber **2** of the preferably body fluid; and a circuit **3** constrained, suitably integral with the absorber 2 and configured to detect the presence of ions and therefore the concentration of a solute in the fluid absorbed by the absorber 2.

The absorber 2 can define a first surface **2a** and a second surface **2b** opposite the first surface 2a and preferably substantially parallel to said first surface 2a.

The first surface 2a can be configured to at least partially come into contact with the body fluid so as to absorb it.

The absorber 2 can further define at least one lateral surface **2c** interposed with the surfaces 2a and 2b.

The absorber 2 can have a porosity substantially equal to at least 40% so as to allow the fluid to be absorbed by the absorber 2, through the first surface 2a, and the circuit 3 to be deposited on the second surface 2b, coating it at least partially without penetrating into the absorber 2 and be absorbed by it. Preferably, the porosity can be almost between 40% and 70% in detail between 50% and 60% and more in detail between 55% and 60%.

The porosity, typical of a structure with small empty spaces, or rather interstices and pores that can be filled by a fluid, can be calculated as the ratio between the volume of the voids and the total volume of a sample of absorber 2.

The absorber 2 can be permeable to body fluid and therefore traversed by it preferably in every direction. It can have a permeability almost equal to at least 5 l/m²/s and in detail substantially between 5 l/m²/s and 25 l/m²/s and more in detail between 10 l/m²/s and 25 l/m²/s and in more detail it reaches between 11 l/m²/s and 15 l/m²/s. It can be substantially equal to 12.6 l/m²/s.

This permeability can be calculated according to ISO 9237/97 applied to the absorber 2.

The absorber 2 can have a weight (or rather a surface density) almost between 80 g/m² and 300 g/m² and in detail practically between 100 g/m² and 200 g/m² and in more detail between 120 g/m² and 150 g/m². It can be substantially equal to 131 g/m².

The grammage can be calculated according to ISO 536 applied to the absorber 2.

The absorber 2 can have a stiffness substantially equal to at least 100 mN and in detail substantially comprised between 100 mN and 500 mN, more in detail between 300 mN and 400 mN. It can be substantially equal to 350 mN.

The stiffness can be calculated according to ISO 2493 applied to absorber 2 (bending angle preferably 15°).

The surfaces 2a and/or 2b can have a roughness almost equal to at least 500 ml/min and in detail substantially between 1000 ml/min and 3000 ml/min and more in detail between 2000 ml/min and 2750 ml/min and more in detail it reaches between 2500 ml/min and 2600 ml/min. Optionally, the first surface 2a can have substantially less roughness than the second surface 2b.

The absorber 2 can be made of hygroscopic material and cellulosic detail (preferably paper such as a paper sheet).

Conveniently, the hygroscopic and in particular cellulosic material of the absorber 2 can be added with stiffening fillers added to the formulation to ensure greater mechanical resistance of the absorber 2 against external actions and/or softening given by the fluid.

Alternatively or in addition it can be added with colouring material and preferably of black colour. Absorber 2 can therefore be black paper.

Said stiffening components can be selected from crosslinking agents and filaments. The absorber 2 can have a thickness, calculated along a sagittal axis perpendicular to the first surface 2a, almost less than 2.5 mm, in detail at 1 mm and more in detail at 0.5 mm. It can be substantially equal to 0.3 mm.

The absorber 2 can have a surface extension, calculated along said first surface 2a, substantially equal to at least 0.1 cm² and in detail between 0.1 cm² and 30 cm² more in detail between 0.3 cm² and 30 cm². It can be substantially equal to 15 cm² or 20 cm².

The circuit 3 can be configured to come into contact with the body fluid only if absorbed by the absorber 2 allowing to detect the concentration of a solute in the fluid absorbed by said absorber 2 and therefore to determine a physiological parameter as a function of said concentration.

It can be made by depositing (such as moulding) an electrically conductive material (such as a polymer and/or an electrically conductive paint) on at least part of the second surface 2b.

The conductive paint may comprise an electrically conductive powder (or rather metallic such as copper, silver and/or gold) or a polymeric dispersion e.g. comprising at least one conductive homopolymer.

In the case of metallic powder, the paint and therefore the circuit 3 can comprise powders of at least a first metal and a second metal having a standard reduction potential greater than the first metal, suitably substantially by at least 1V. For example, the paint may have a silver powder content substantially between 15% and 20% and a copper powder content substantially between 3% and 5%.

The circuit 3 can comprise at least one electrode and in detail several electrodes. Preferably it comprises a first electrode **31**, a second electrode **32**, a third electrode **33** and optionally a fourth electrode **34**.

More preferably the circuit 3 can be an OECT and therefore the first electrode 31 can identify the drain, the second electrode 32 can identify the source, the third electrode 33 can identify the gate and the eventual fourth electrode 34 can identify an additional gate.

Each electrode can comprise a head and suitably a branch protruding from said head towards a second electrode and defining an axis of preferred extension.

Head and branch can be of the same material.

The electrode heads can have the same shape. They can be circular of suitably equal diameter. Said diameter can be practically less than 3 cm in detail substantially between 2 cm and 0.2 cm and more in detail between 1 cm and 0.5 cm. It is preferably substantially equal to 0.85 cm.

The branches of the electrodes can have the same length.

The length of the branches, calculated along said axis of preferred extension, can be almost less than 3 cm in detail substantially between 2 cm and 0.2 cm and more in detail between 1 cm and 0.5 cm.

The width of the branches, normally calculated for said axis, can be almost less than 5 mm, in particular 3 mm, almost between 3 mm and 1 mm and in detail almost equal to 2 mm.

The axes of preferred extension of the branches of the first 31 and of the second electrode 32 can be practically parallel to each other and almost coincident in detail. The axis of preferred extension of the branch of the third electrode 33 can be substantially perpendicular to that of the branch of the first electrode 31.

The axes of preferred extension of the branches of the third 33 and fourth electrode 34 can be substantially parallel to each other and substantially coincident in detail.

The axes of preferred extension of the electrode branches can be incised in a single point. The electrodes 31, 32, 33 and 34 thus form a cross circuit 3 in which the heads identify the ends of said cross.

Preferably the electrodes 31, 32, 33 and 34 are not in contact with each other.

The first 31 and the second electrode 32 are spaced apart by a distance of at least 0.5 cm in detail practically comprised between 0.5 cm and 3 cm and precisely between 1 cm and 2 cm. Said distance can be substantially equal to 1.5 cm.

The third electrode 33 is at a distance from the electrodes 31 and 32 practically less than 5 mm, in detail at 3 mm and to be precise, practically between 2 mm and 1 mm.

The fourth electrode 34 is at a distance from the other electrodes almost less than 5 mm in detail at 3 mm and preferably between 2 mm and 1 mm.

Third 33 and fourth electrodes 34 are at a distance of at least 0.2 cm in detail substantially comprised between 0.2 cm and 2 cm and precisely between 0.4 cm and 1 cm. Said distance can be substantially equal to 0.6 cm.

In this document the distance of an electrode from another element is calculated along the axis of preferred extension of the branch of said electrode.

The third electrode 33 is configured to detect the concentration of at least one solute in the body fluid. For this purpose, it can be activated, for example, by coating at least the branch. For example, the solute is one or more of sodium, calcium and potassium and therefore the third electrode 33 can be coated in a conductive metal material (such as nickel, copper, gold and/or silver) or conductive polymeric material (such as polypyrrole, polyaniline, PEDOT-PSS).

The fourth electrode 34 is configured to detect the concentration in the body fluid of at least one additional solute in detail different from the solute of the third electrode 33. The additional solute is for example one or more of cortisol, adrenaline and the fourth electrode 34 can be coated with enzymes, MIP (Molecularly Imprinted Polymer), supramolecular receptors (such as cavitands such as β-cydodextrin).

The circuit 3 can comprise an active connection **35** of two electrodes and in detail of the first electrode 31 to the second 32.

The connection 35 can be placed between the electrodes and the absorber 2. It is deposited on the second surface 2b.

The active connection 35 can define a contact between the first 31 and the second electrode 32 (in detail between their branches) so as to have a current passage between the electrodes 31 and 32.

The active connection 35 may not be in contact with the third electrode 33. The distance between them can be almost less than 5 mm in detail to 3 mm and to be precise substantially between 2 mm and 1 mm.

It may not be in contact with the fourth electrode 34. Their distance may be practically less than 5 mm in detail at 3 mm and to be precise substantially between 2 mm and 1 mm.

The active connection 35 can be of electrically conductive material in detail sensitive to the ions of the solute and preferably of the additional solute. Said material sensitive to said ions can be an electrically conductive polymer such as said paint preferably with particles in emulsion of a conductive polymer such as polypyrrole, polyaniline, PEDOT-PSS.

The electrochemical physiological sensor 1 can comprise an insulator **4** defining a housing chamber of at least absorber 2 and circuit 3.

The insulator 4 defines at least one opening for the passage of body fluid between the exterior and the chamber and in detail between the exterior and the absorber 2.

It can define at least one inlet opening **4a** of the body fluid into the chamber and at least one outlet opening **4b** of the body fluid from said chamber so as to generate a flow of body fluid that crosses the absorber 2 and enters the insulator in detail 4 from the inlet opening 4a, is absorbed by the absorber 2 and emerges from the outlet opening 4b.

Preferably, the insulation 4 comprises a single inlet opening 4a.

The inlet opening 4a can overlap (in this document the term overlap is to be understood with respect to the aforementioned sagittal axis) to the active connection 35.

It can be in correspondence with the first surface 2a and therefore on the opposite side to the connector 35 so that the body fluid comes into contact with the connector 35 only when absorbed by the absorber 2.

The inlet opening 4a can have an almost equal section and in detail almost greater and in more detail at least double that of the connector 35.

The at least one outlet opening 4b can face the absorber 2 preferably in correspondence with the lateral surface 2c. Then the fluid enters the absorber 2 from the first surface 2a, thanks to the inlet opening 4a, and then exits from the lateral surface 2c thanks to the outlet opening 4b.

The insulator 4 can define a first base surface **4c** facing (in detail in contact with the first surface 2a) and comprising the inlet opening 4a.

The insulator 4 can define a second base surface **4d** facing and in detail in contact with the second surface 2b and circuit 3.

The base surface 4d can be without openings.

The insulator 4 can define a perimeter surface **4e** connecting said base surfaces 4c and 4d and preferably comprising one or more outlet openings 4b.

The perimeter surface 4e can be overlapped on the lateral surface 2c.

The insulation 4 can comprise several outlet openings 4b suitably equally spaced along the perimeter surface 4e.

The insulator 4 can comprise a first layer **41** defining the first base surface 4c and therefore the inlet opening 4a and a second layer **42** defining the second base surface 4d.

The layers 41 and 42 are configured to be constrained to each other, suitably in correspondence with the perimeter, delimiting the insulation chamber 4 and defining the perimeter surface 4e.

They have a surface extension substantially at least equal to and optionally almost greater than that of the absorber 2.

The perimeter surface 4e can comprise a junction profile between the layers 41 and 42 interrupted by at least one outlet opening 4b. In detail, the surface 4e can comprise several outlet openings 4b suitably equally spaced.

The joining profile can be made by combining the layers 41 and 42 together and suitably the absorber 2.

In particular, the profile can be made by melting at least one of the layers (in detail both layers 41 and 42) suitably in correspondence with the its perimeter. More particularly, it can lead to impregnating the absorber 2 (suitably in correspondence with at least part of the perimeter) with the casting material of at least one of the layers and preferably both layers 41 and 42. The joining profile can thus be made from the junction of the layers 41 and 42 and of the absorber 2 suitably in correspondence with its perimeter.

It is evident that this process alters the functionality of the absorber substantially only in correspondence with its perimeter which becomes hermetic as it is impregnated with the material of the layers 41 and/or 42. At the outlet openings 4b the material of the layers 41 and/or 42 it is not melted and therefore the absorber 2 does not absorb material of the layers 41 and/or 42 maintaining its own characteristics and remains non-hermetic allowing the body fluid to escape.

The joint profile can be made by welding such as heat sealing or ultrasound.

The layers 41 and 42 and therefore the insulation 4 can be electrically insulating.

The layers 41 and 42 and therefore the insulation 4 can be impermeable to the body fluid so that the body fluid only passes through the openings 4a and 4b.

Advantageously, the layers 41 and 42 and therefore the insulation 4 are made of an electrically insulating and impermeable material such as non-woven fabric or polypropylene.

The electrochemical physiological sensor 1 can comprise at least one film **5**.

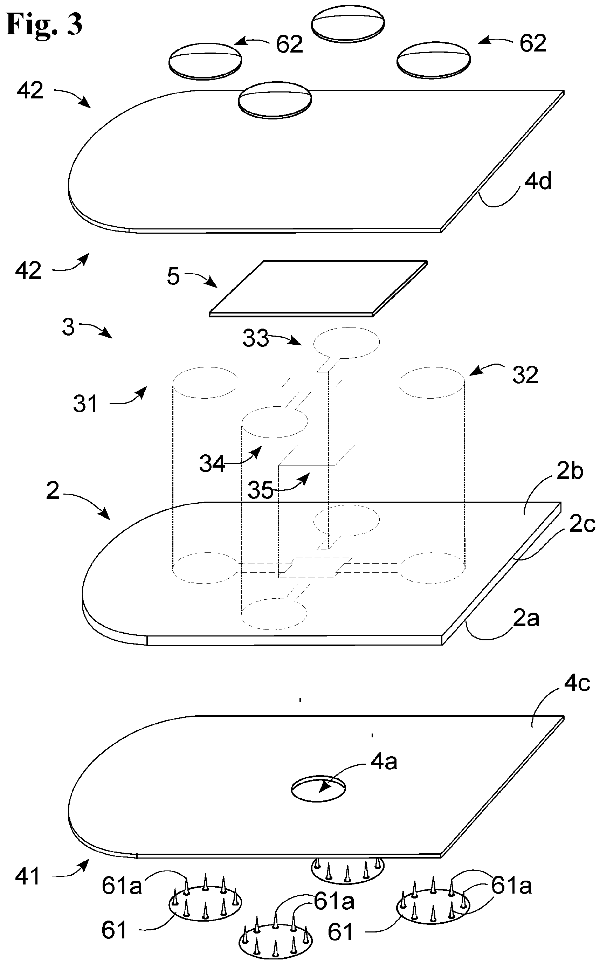

In particular, it can comprise a film 5 placed between insulator 4 (in detail the second layer 42) and at least part and in detail the whole of the circuit 3 (as shown in Fig. 3).

In detail, the film 5 can cover the active connection 35 and at least the branches of the electrodes and suitably the entire circuit 3. It can therefore be identified in a sheet without holes/openings. Alternatively, the film 5 can be a frame of the active connection 35 which is not superimposed thereon.

Preferably the film 5 can adhere to at least part and in detail the whole of the circuit 3 and preferably to at least part of the absorber 2 (in detail of the second surface 2b) enclosing the circuit 3 between the absorber 2 and the film 5. The circuit 3 is therefore isolated and not in contact with air or other agents other than the fluid and which may oxidize or in any case deteriorate the circuit 3.

As an alternative or addition, the sensor 1 can comprise a film 5 between the first surface 2a and the first layer 41. Preferably the film 5 is a frame of the entrance opening 4a not overlapped thereon.

The film 5 can be an electrically insulating material.

It is adhesive. It therefore adheres to at least part of the absorber 2 and/or of the circuit 3.

The electrochemical physiological sensor 1 can comprise at least one connector **6** for each electrode; and preferably a control board **7** of the circuit 3.

Each connector 6 defines a data passage between board 7 and circuit 3.

The connector 6 can be configured to be constrained externally to the insulation 4 by means of snap fastening, for example. In detail, the connector 6 can comprise a first body **61** and a second body **62** configured to be placed on the opposite side to the insulator 4 and preferably engage, preferably, to the first body 61, enclosing the insulator 4 together.

The bodies 61 and 62 can be circular in detail with a diameter almost less than 3 cm, more in detail substantially between 2 cm and 0.2 cm and in more detail still between 1 cm and 0.5 cm. It can be almost equal to 0.85 cm or 0.8 cm.

They can have the same shape and preferably the same dimensions as the electrode heads.

The first body 61 can comprise at least one tooth **61a** and the second body 62 can comprise, for each tooth 61a, an engagement seat for the tooth 61a.

The tooth 61a is configured to cross and engage with the first layer 41, an electrode, the absorber 2, the second layer 42 and finally the second body 62.

Preferably the first body 61 can comprise more teeth 61a (for example five) configured to constrain itself to an electrode along the perimeter of the head.

It can be seen how the connector 6, by clamping together the first layer 41, circuit 3, absorber 2, second layer 42, identifies a discharge point for external loads.

The board 7 is external to the insulator 4.

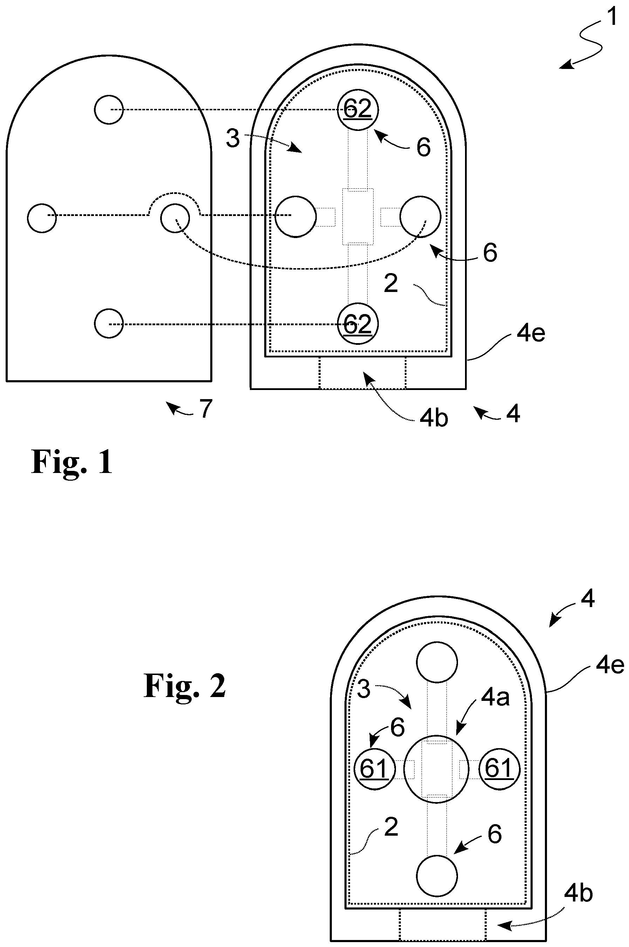

It can be in data connection, for example wired (see Fig. 1), with each connector 6 suitably with the second body 62.

The board 7 can be constrained in a resolvable way to the connector 6.

The board 7 can comprise a parameter database associating the value of a physiological parameter (such as the degree of hydration) to at least one concentration of one or more solutes.

It may comprise an absorber database comprising characteristics of the absorber 2 such as porosity, permeability or others described above.

The board 7 can comprise a storage memory of said databases and/or of the data acquired by the sensor 1.

The board 7 can comprise an antenna configured to allow a (wireless) data exchange between sensor 1 and an external device for interfacing with the user.

The board 7 can be configured to control circuit 3. In detail, it is configured to apply a potential, suitably independently, to one or more electrodes, defining one or more acquisition configurations for circuit 3 (therefore sensor 1) of a current between a pair of electrodes.

In each acquisition configuration a potential difference is defined between two electrodes and in detail between first 31 and second electrode 32 suitably for an activity time.

The potential difference can be defined with the first electrode 31 discharged (at zero potential) and in detail only the second electrode 32 positively charged.

The activity time can be the same for the various configurations.

The activity time can be substantially less than 60" in detail almost between 20" and 40" and in more detail almost equal to 30".

The board 7 can define a first acquisition configuration by defining a first potential difference between the first 31 and the second electrode 32.

The first potential difference can be almost lower than 1 V in detail at 0.5 V in more detail at 0.2 V. It can be substantially equal to 0.1 V.

In this configuration the third electrode 33 and the eventual fourth 34 are discharged. The board 7 can define a second acquisition configuration by defining a second potential difference between the first 31 and the second electrode 32 and a charge potential of the third electrode 33.

The second potential difference can be almost less than 1 V in detail at 0, 5 V more in detail at 0.2 V. It can be substantially equal to 0.1 V.

The second potential difference can be equal to the first.

The charging potential can be positive.

It can be substantially greater than the second potential difference.

The charging potential can be substantially lower than 1 V in detail to 0.7 V more in detail substantially between 0.5 V and 0.2 V. It can be substantially equal to 0.3 V.

It is evident that in this configuration since the third electrode 33 is charged and the first electrode unloaded, there is a potential difference between them.

In the second configuration, the fourth electrode 34 can be discharged.

The board 7 can define a third acquisition configuration by defining a third potential difference between first 31 and second electrode 32 and a third charging potential of the fourth electrode 34.

The third potential difference can be substantially less than 1 V in detail at 0,5 V in more detail to 0.2 V. It can be substantially equal to 0.1 V.

The third potential difference can be equal to the first.

The third charge potential can be positive.

It can be almost greater than the third potential difference.

The third charge potential can be substantially lower than 1 V in detail at 0.7 V more in detail at 0.5 V. It can be substantially equal to 0.3 V.

It is evident that in this third configuration, since the fourth electrode 34 is charged and the first electrode unloaded, there is a potential difference between them.

In the third configuration, the third electrode 33 can be discharged.

The electrochemical physiological sensor 1 can comprise a power supply of the sensor 1 such as a battery.

The electrochemical physiological sensor 1 can comprise a container defining a housing for the aforementioned components of the sensor 1.

The invention comprises a new method for making an electrochemical physiological sensor 1 in accordance with what is described above in structural terms.

The realization process can comprise a realization phase the circuit 3 on the absorber 2 in which there is the deposit (moulding) of at least one electrically conductive material on the absorber 2, making the active connection 35 and the electrodes of the circuit 3.

In order to favour the drying of the electrically conductive material, the deposit can be made on the absorber 2 at a heating temperature practically between 80°C and 130°C and in detail substantially equal to 120°C.

The realization phase can comprise a first sub-phase of depositing on part of the second surface 2b of a first electrically conductive material creating the active connection 35 and a second sub-phase of depositing a second electrically conductive material creating the electrodes 31, 32, 33 and optionally 34.

In said sub-phases, the deposit of said materials can be carried out by moulding in detail, be it by spraying, screen printing, screen printing or pad printing.

In the first sub-phase a first adhesive mask can be used which reproduces in negative the active connection 35.

In the first sub-phase the deposit can be made by heating the absorber 2 to a temperature substantially equal to said heating temperature.

In the second deposition sub-phase it can be used using a second adhesive mask reproducing the electrodes in negative.

In the second sub-phase, the deposit can be carried out by heating the absorber 2 to a temperature substantially equal to said heating temperature.

The second material is deposited on part of the active connection 35 and on part of the second surface 2b. In detail, the second material defining the first 31 and second electrode 32 is deposited on part of the active connection 35 and of the second surface 2b, while that defining the third electrode 33 and possible fourth 34 is deposited only on part of the second surface 2b.

The first material can be different or preferably the same as the second material.

The first material can be a polymeric paint described above. Preferably it is sensitive to the ions of the solute and/or of the additional solute and, in detail, it can be an electrically conductive polymer such as said paint preferably with emulsion particles of a conductive polymer such as polypyrrole, polyaniline, PEDOT-PSS.

The second material can be a metallic paint comprising metallic powders of, for example copper, silver and/or gold as described above.

The realization process may comprise an incapsulating phase at least absorber 2 and circuit 3 in the insulation 4.

The incapsulating phase may comprise a covering sub-phase in which a film 5 is constrained to the circuit 3 and to the absorber 2 (in detail to the second surface 2b) covering them at least partially.

Said film 5 may not cover the active connection 35. Preferably it covers the active connection 35.

Optionally, the storage phase can comprise a protection sub-phase in which a film 5 is constrained to the absorber 2 and in detail to the first surface 2a.

Said film 5 may not cover the inlet opening 4a.

The incapsulating phase can comprise a sub-step of insertion into the insulation 4 of the assembly (absorber 2, circuit 3 and optionally at least one film 5).

In the insertion sub-phase, said assembly is placed between the first layer 41 and the second 42 with the first surface 2a and the second surface 2b respectively proximal and to be precise in contact with the first base surface 4c and second 4d.

Once this sub-phase is closed, the incapsulating phase can comprise a constraint sub-phase of the first layer 41 to the second 42, creating a perimeter surface 4e.

In this sub-phase a perimeter surface 4e is created comprising a junction profile between the layers 41 and 42 interrupted by at least one outlet opening 4b and in detail by several openings 4b suitably equally spaced apart.

The junction profile is made along (suitably only part) the perimeter of layers 41 and 42. It is made by suitably merging at least one of the layers (in detail both layers 41 and 42) which is therefore absorbed and impregnates the perimeter. absorber 2.

The perimeter of the absorber 2, impregnated by said layers 41 and/or 42, therefore defines the junction profile suitably hermetic and in detail insulating at least electrically.

The profile delimits the chamber containing the absorber 2.

The joint profile can be obtained by welding (such as heat sealing or ultrasound).

The realization process can comprise a connecting phase at least one connector 6 to the circuit 3 and, to be precise, of a connector 6 to each electrode suitably in correspondence with the head only.

In this phase, a first body 61 is placed in correspondence with the first layer 41 and a second body 62 in correspondence with the second layer 42. Therefore, the bodies 61 and 62 are pressed and constrained to each other causing the teeth 61a to penetrate layers 41 and 42, absorber 2 and circuit 3 locking them together.

The teeth 61a of each body 61 penetrate a head (in detail along its perimeter) allowing an electrical signal to pass between the connector 6 and the electrode.

The realization process can comprise a connection phase of the board 7 to the circuit 3 through said at least one connector 6.

Optionally, in the connection phase the power supply can be connected to the rest of the sensor 1 through said board 7.

The realization process can finally comprise a housing step in the board container 7, insulator 4 (with the aforementioned assembly inside it) and optionally a power supply.

The operation of the electrochemical physiological sensor 1 introduces a new procedure for calculating the concentration of at least one solute in a body fluid configured to be implemented by sensor 1.

The calculation procedure can be controlled by board 7.

It can include a setting phase for the sensor 1 in which circuit 3 is in the first acquisition configuration and the setting current between first 31 and second electrode 32 is determined in a dry condition, i.e. without fluid absorbed by the absorber 2.

The measurement of each setting current is stored in the board memory 7.

The calculation procedure may comprise a wetting step in which the absorber 2 absorbs a body fluid passing from the dry to the wet condition.

In this phase a body fluid is introduced through the inlet opening 4a into the chamber and is absorbed by the absorber 2. The fluid thus wets at least the active connection 35 (and in detail at least part of the electrode branches) and can escape from the absorber 2 from the outlet opening 4b.

Body fluid can be a sample collected by a user previously.

Alternatively, the at least one sensor 1, for example integrated in a garment worn by the user, has the first layer 41 and therefore the inlet opening 4a in contact with the body fluid which can thus be absorbed by the absorber 2 through said inlet opening 4a.

The calculation procedure can comprise at least one evaluating phase of the concentration of at least one solute in the body fluid.

Preferably it comprises several evaluation steps so as to carry out a continuous monitoring of said solute concentration.

The evaluation phase may comprise a first measurement sub-phase in which the circuit 3 is in the first acquisition configuration and a first current is determined between the first 31 and the second electrode 32 in a wet condition and in detail an additional first current between the first electrode 31 and third electrode 33 in a wet condition.

The first measurement sub-phase can have a duration equal to the activity time.

In the first measurement sub-phase only the final value of the first current can be measured (final in this document is to be referred respectively to the instant prior to the end of the sub-phase).

In said first sub-phase only the final value of the additional first current can be measured.

The evaluation phase can comprise a second measurement sub-phase in which the circuit 3 is in the second acquisition configuration and a second current is determined between the first electrode 31 and the second electrode 32 in a wet condition and in detail an additional second current between the first electrode 31 and third electrode 33 in a wet condition.

The second measurement sub-phase can have a duration equal to the activity time.

In the second measurement sub-phase, only the final value of the second current can be measured.

In said second sub-phase only the final value of the additional second current can be measured.

It can be seen that the first and second currents are different from each other thanks to the third electrode 33 which is discharged in the first configuration and charged in the second.

The evaluation phase can comprise a sub-phase of determining the concentration of the solute as a function of the currents determined in the measurement sub-phases, in the setting phase and preferably of the absorber database.

In detail, in this sub-phase the amount of fluid in the absorber 2 can be determined as a function of additional second current, first current, second current, setting current and preferably of the additional first current and/or at least the porosity of the absorber. 2. Preferably it is inversely proportional to the second current and to the setting current and directly to the additional second current and preferably to the additional first current and/or to at least the porosity of the absorber 2.

The solute concentration can be a function of the additional second current, the first stream, the amount of fluid in the absorber 2, the second stream, the setting stream and preferably the additional first stream and/or the porosity of the absorber. In detail, it can be linearly dependent on the additional second current and preferably on the additional first current, inversely proportional to the quantity of fluid in the absorber 2, directly proportional to the first current, to the second current, to the setting current and preferably to the porosity of the absorber.

In the determination sub-phase, the concentration of the solute can be a function of the parameters database and/or the absorber database.

It should be noted that the evaluation phase can include, at the end of each second measurement sub-phase, a discharge sub-phase in which all the electrodes are discharged. Consequently, each evaluation phase can include, suitably in order, a first measurement sub-phase, a second measurement sub-phase, a discharge sub-phase and therefore a determination sub-phase.

Once the evaluation phase has been completed, the calculation procedure may include a signalling phase in which the board 7 sends the solute concentration value to an external device.

The calculation method can comprise a detecting phase of the concentration of at least one additional solute of said body fluid. In detail, it can comprise several additional evaluation phases so as to have a continuous monitoring of the concentration of the additional solute.

The additional solute can be cortisol, adrenaline or other substance present in the fluid and preferably different from that determined in the evaluation phase.

The detection phase can comprise a first relief sub-phase in which the circuit 3 is in the first acquisition configuration and a first current is determined between the first electrode 31 and the second electrode 32 in a wet condition and an additional first current between the first 31 and the fourth electrode 34 in wet condition.

The first survey phase can have a duration equal to the activity time.

In the first survey sub-phase, only the final value of the first current can be measured.

In said first sub-phase only the final value of the additional first current can be measured.

The detection phase can comprise a second survey sub-phase in which the circuit 3 is in the third acquisition configuration and a third current is determined between the first electrode 31 and the second electrode 32 in a wet condition and an additional third current between the first electrode 31 and the fourth electrode 34 in a wet condition.

The second survey phase can have a duration equal to the activity time.

In the second survey sub-phase, only the final value of the third current can be measured.

In said second sub-phase only the final value of the additional third current can be measured.

It can be seen that the first and third currents are different from each other thanks to the fourth electrode 34 which is discharged in the first configuration and charged in the third.

The detection phase may comprise a sub-phase of computation of the concentration of the additional solute as a function of the currents determined in the relevant sub-phases, in the setting phase and preferably of the absorber database.

In detail, in the calculation sub-phase the amount of fluid in the absorber 2 can be determined as a function of first current, additional third current, third current, setting current and preferably of additional first current and/or at least the porosity of absorber 2. Preferably it is inversely proportional to the third current, to the first current and to the setting current and directly to the additional third current and preferably to the additional first current and/or to at least the porosity of the absorber 2.

The solute concentration can be a function of the additional first stream, the first stream, the additional third stream, the amount of fluid in the absorber 2, the second stream, the setting stream and preferably the additional first stream and/or the porosity of the absorber. In detail, it can be linearly dependent on the additional third current and preferably on the additional first current, inversely proportional to the quantity of fluid in the absorber 2, directly proportional to the third current, to the first current, to the setting current and preferably to the porosity of the absorber.

It should be noted that the detection phase can include, at the end of each second survey sub-phase, an additional discharge sub-phase in which all the electrodes are discharged. Consequently, each evaluation phase can include, suitably in order, a first survey sub-phase, a second survey sub-phase, an additional discharge sub-phase and a calculation sub-phase.

Once the detection phase has been completed, the calculation procedure may comprise a warning phase in which the board 7 sends the value of the concentration of the additional solute to an external device.

It should be noted that the various passages of the circuit 3 in the various acquisition configurations are obviously controlled by the board 7.

The electrochemical physiological sensor 1, the relative manufacturing procedure and the calculation procedure according to the invention achieve important advantages.

In fact, compared to known physiological sensors, the electrochemical physiological sensor 1 allows to perform an analysis of the body fluid of high quality and in particular a detection of the concentration of a solute in the body fluid.

This aspect is determined by the particular specifications of the absorber 2 which allows to perform an extremely precise deposit of the circuit 3. In detail, one of the peculiar characteristics of the absorber 2 which made it possible to obtain this is to be identified in the porosity which allows, as discovered by the owner, the electrically conductive polymer to be deposited without being absorbed by the absorber and therefore without deforming due to said absorption.

It should be noted that the characteristics of the absorber 2 are also optimal for the absorption of the body fluid and for its resistance to stress and wear.

This high quality of analysis is also given by the particular calculation procedure which, by exploiting the various acquisition configurations in an innovative way, allows to exploit the electrodes in an optimal way and therefore to have precise measurements of the current between said electrodes.

Another advantage is given by the particular arrangement of the openings 4a and 4b which allows to obtain a flow of body fluid through the absorber ensuring a continuous change of fluid.

This aspect is due to the position of at least one outlet opening 4b (in detail more openings 4b) along the perimeter surface 4e which allows to have an outlet opening 4b facing downwards so as to exploit gravity to control the outflow of fluid from the absorber 2. For example, in fact, the sensor can be worn by placing itself in contact with the skin of an arm/chest of a user who, having vertical arm/chest positions during his activity, allows one of the exit openings to be proximal to a point of sensor 1 with a lower gravitational potential.

Another advantage is that the film 5 prevents the air or other gases entering the sensor 1 (to be precise in the housing chamber defined by the insulator 4) from coming into contact with the circuit 3, oxidizing it or in any case altering/deteriorating its performance.

An advantage is in the use of the aforesaid electrically conductive powder which allows to have a circuit 3 comprising metals with different reduction potential so as to concentrate the oxidation only on one of them.

Another advantage is given by the possibility of alternatively exploiting the third electrode 33 and the fourth 34 and therefore of carrying out analyses of different solutes and therefore of different biological parameters.

A not secondary advantage is given by the choice of the connectors 6 which, thanks to the perimeter teeth 61a at the head of the electrodes, guarantees an optimal signal passage.

An important advantage is given by the calculation procedure and in particular by the definition of the various acquisition configurations that allow the concentration to be precisely determined.

In fact, for example, the adoption of the calculation of the setting current allows to highlight any dry conductivity, i.e. due to the peculiar characteristics of the sensor 1 such as those given by machining inaccuracies and/or by the geometry of the circuit 3 and/or the absorber 2.

Furthermore, the calculation procedure and therefore the sensor 1 allow to calculate the amount of body fluid absorbed by the sensor 1 and therefore determine the flow of body fluid passing through the absorber 2.

Consequently, the calculation procedure and therefore the sensor 1 allows to analyse the concentration of the solute and/or the additional solute regardless of the wetting conditions of the absorber 2a and therefore regardless of whether the active areas of the circuit 3 (i.e. the porous area of the absorber 2 corresponding to the active connection 35 and that/those between active connection 35 and electrode 33 or 34 are partially or integrally filled with the body fluid). Therefore, the calculation procedure takes into account how wet the active areas of the sensor are and the difference in wetting between said areas.

This high precision is also determined by the exploitation for the calculation of the concentration of the calculation of the current between the first electrode and the third electrode 33 and/or the fourth electrode 34 which allows to evaluate the concentration of the solute also as a function of how wet the absorber 2 is and therefore of the body fluid content in the absorber 2.

A further advantage is given by the sensor 1 which allows to have lower costs and production times and at the same time a high duration and reliability.

The invention is susceptible of variants falling within the scope of the inventive concept defined by the claims.

For example, the absorber 2 can have a porosity substantially between 4000 ml/min and 8000 ml/min, in detail between 6000 ml/min and 7000 ml/min and more in detail almost equal to 6280 ml/min. Said porosity values are calculated according to the Bendtsen method (ISO 5636/3 applied to absorber 2).

In this context, all the details can be replaced by equivalent elements and the materials, shapes and dimensions can be any.

## Claims

1. Electrochemical physiological sensor (1) configured to contact a body fluid and comprising
- an absorber (2) of said body fluid defining a first surface (2a) configured to come into contact at least partially with said body fluid and a second surface (2b) opposite to said first surface (2a);
- a circuit (3) deposited on said second surface (2b) and configured to come into contact with said body fluid when absorbed by said absorber (2) so as to allow to detect the concentration of a solute in said body fluid absorbed by said absorber (2); and **characterized by**
- said absorber (2) has a porosity substantially between 40% and 70% so as to allow said body fluid to be absorbed by said absorber (2) and said circuit (3) to be deposited covering at least part of said second surface (2b) without penetrating into said absorber (2).

2. Electrochemical physiological sensor (1) according to claim 1, wherein said porosity of said absorber (2) is substantially comprised between 40% and 50%.

3. Electrochemical physiological sensor (1) according to at least one preceding claim, wherein said absorber (2) is made of paper material added with stiffening components of said paper material; and wherein said stiffening components are selected from film forming agents and filaments.

4. Electrochemical physiological sensor (1) according to at least one preceding claim, wherein said circuit (3) comprises a first metal and a second metal having a standard reduction potential greater than said first metal.

5. Process for manufacturing an electrochemical physiological sensor (1) **characterized by** comprising a manufacturing phase wherein an electrically conductive material is deposited on an absorber (2) having a porosity substantially between 40% and 70% so as to allow said body fluid to be absorbed by said absorber (2) and to said electrically conductive material and therefore to said circuit (3) to be deposited covering at least part of said second surface (2b) without penetrating into said absorber (2).

6. Implementation method according to the preceding claim, wherein said circuit (3) comprises electrodes (31, 32, 33, 34) and an active connection (35) of two of said electrodes (31, 32, 33, 34); and in which said realization step comprises a first sub-phase of depositing on said absorber (2) of a first electrically conductive material realizing said active connection (35) and a second sub-phase of depositing a second electrically conductive material realizing said electrodes (31, 32, 33, 34) on part of said active connection (35) and on part of said absorber (2); and wherein in each of said deposition sub-phases said second electrically conductive material comprises a first metal and a second metal having a standard reduction potential greater than said first metal; and wherein said first electrically conductive material comprises an electrically conductive polymer.

7. Electrochemical physiological sensor (1) configured to contact a body fluid and comprising
- an absorber (2) of said body fluid defining a first surface (2a) configured to come into contact at least partially with said body fluid and a second surface (2b) opposite to said first surface;
- a circuit (3) deposited on said second surface (2b) and configured to come into contact with said body fluid when absorbed by said absorber (2) so as to allow at least one solute of said body fluid to be detected as a function of said absorbed body fluid from said absorber (2);
**characterized by** comprising
- an insulator (4) defining a chamber for housing said absorber (2) and said circuit (3) and defining
∘ a first base surface (4c) superimposed on said first surface (2a),
∘ a second base surface (4d) superimposed on said second surface (2b) and on said circuit (3),
∘ a perimeter surface (4e) connecting said base surfaces (4c, 4d);
**by** that
- said first base surface (4c) comprises at least one inlet opening (4a) configured to place said first surface (2a) in contact with said body fluid allowing said body fluid to enter said chamber and be absorbed by said absorber (2);
**and the fact** that
- said perimeter surface (4e) comprises at least one outlet opening (4b) of said body fluid from said absorber (2) and from said chamber thus defining, together with said inlet opening (4a), a path of said body fluid which enters said chamber through said first surface (2a), passes through said absorber (2) and exits from said chamber through said outlet opening (4b).

8. Electrochemical physiological sensor (1) according to claim 7, comprising an electrically insulating film 5 and sandwiched between said insulator (4) and at least part of said circuit (3); and in which said film (5) is adhesive and adheres to one of said insulator (4) and said circuit (3).

9. Electrochemical physiological sensor (1) according to at least one claim 7-8, wherein said insulator (4) comprises a first layer (41) defining said first base surface (4c) and therefore said inlet opening (4a) and a second layer (42) defining said second base surface (4d); and wherein said perimeter surface (4e) comprises a junction profile between said layers (41, 42) interrupted by said outlet opening (4b).

10. Electrochemical physiological sensor (1) according to claim 9, wherein said insulator (4) comprises a plurality of said at least one outlet opening (4b) equally spaced apart and therefore said junction profile is interrupted by said plurality of said openings outlet (4b).

11. Electrochemical physiological sensor (1) according to at least one claim 9-10, comprising a board (7) of said circuit (3) external to said chamber and at least one connector (6) connecting said board (7) and said circuit (3) through said insulator; wherein said connector (6) comprises a first body (61) configured to engage at least said first layer (41) and a second body (62) configured to engage at least said second layer (42); and in which said bodies (61, 62) are configured to mutually constrain each other by clamping together at least said insulator (4), said absorber (2) and said circuit (3).

12. Process for manufacturing an electrochemical physiological sensor (1) according to at least one claim 7-10; said procedure comprising
- a making phase of said circuit (3) on said absorber (2);
**characterized by comprising** an encapsulation phase comprising
- an insertion sub-phase in which said absorber (2) and said circuit (3) are arranged between said first layer (41) and said second layer (42); and
- a constraining sub-phase of said first layer (41) to said second layer (42) realizing a junction profile delimiting said chamber of said circuit (3) and said absorber (2) and interrupted by at least one outlet opening (4b).

13. Manufacturing process according to claim 11, wherein in said constraining sub-phase said junction profile is made by perimeter welding of said layers (41, 42).

14. Electrochemical physiological sensor (1) configured to contact a body fluid and comprising
- an absorber (2) of said body fluid;
- a circuit (3) configured to come into contact with said body fluid when absorbed by said absorber (2) so as to allow determining the concentration of a solute in said body fluid absorbed by said absorber (2);
- said circuit (3) being an OECT and comprising a first electrode (31), a second electrode (32) and a third electrode (33);
**characterized by** comprising a control board (7) for said electrochemical physiological sensor (1) defining for said circuit (3)
- a first acquisition configuration in which a potential difference is defined between said first electrode (31) and said second electrode (32) and said third electrode (33) is discharged;
- a second acquisition configuration in which a second potential difference is defined between said first electrode (31) and said second electrode (32) and defining a charge potential of said third electrode (33); and
and **by that** said board (7) is configured to operate in said physiological electrochemical sensor (1) a method for calculating the concentration of a solute in said body fluid comprising
- a setting phase comprising an estimation sub-phase in which said circuit (3) is in said first acquisition configuration and the setting current between said first electrode (31) and said second electrode (32) in dry condition is determined;
- at least one evaluating phase said concentration of at least one solute in said body fluid; said evaluation phase comprises
∘ a first measurement sub-phase in which said circuit (3) is in said first acquisition configuration and a first current is determined between said first electrode (31) and said second electrode (32) in a wet condition;
∘ a second measurement sub-phase in which said circuit (3) is in said second acquisition configuration and a second current is determined between said first electrode (31) and said second electrode (32) in said wet condition and an additional second current between said first electrode (31) and said third electrode (33) in said wet condition;
∘ a determination sub-phase in which the concentration of a solute in said body fluid is determined as a function of said first current, said second current, said additional second current and said setting current.

15. Process for calculating the concentration of a solute in a body fluid comprising an electrochemical physiological sensor (1) configured to come into contact with a body fluid; said electrochemical physiological sensor (1) comprising
- an absorber (2) of said body fluid;
- a circuit (3) configured to come into contact with said body fluid when absorbed by said absorber (2) so as to allow determining the concentration of a solute in said body fluid absorbed by said absorber (2);
- said circuit (3) being an OECT and comprising a first electrode (31), a second electrode (32) and a third electrode (33);
**characterized by** comprising a control board (7) for said electrochemical physiological sensor (1) defining for said circuit (3)
- a first acquisition configuration in which a potential difference is defined between said first electrode (31) and said second electrode (32) and said third electrode (33) is discharged;
- a second acquisition configuration in which a second potential difference is defined between said first electrode (31) and said second electrode (32) and defining a charge potential of said third electrode (33);
**by the fact that** said calculation procedure comprises
- a setting phase comprising an estimation sub-phase in which said circuit (3) is in said first acquisition configuration and the setting current between said first electrode (31) and said second electrode (32) in dry condition is determined;
- at least one step of evaluating said concentration of at least one solute in said body fluid; said evaluation phase comprises
∘ a first measurement sub-phase in which said circuit (3) is in said first acquisition configuration and a first current is determined between said first electrode (31) and said second electrode (32) in a wet condition;
∘ a second measurement sub-phase in which said circuit (3) is in said second acquisition configuration and a second current is determined between said first electrode (31) and said second electrode (32) in said wet condition and an additional second current between said first electrode (31) and said third electrode (33) in said wet condition;
∘ a determination sub-phase in which the concentration of a solute in said body fluid is determined as a function of said first current, said second current, said additional second current and said setting current.

16. Calculation method according to at least one claim 5-7, in said circuit (3) comprising a fourth electrode (34); wherein in said first acquisition configuration said fourth electrode (34) is discharged; wherein said board (7) defines for said circuit (3) a third acquisition configuration in which a third potential difference is defined between said first electrode (31) and said second electrode (32) and defining an additional charge potential of said fourth electrode (34); and wherein said calculation procedure comprises:
- at least one detecting phase said concentration of at least one additional solute in said body fluid; said detection step comprises
∘ a first relevant sub-phase in which said circuit (3) is in said first acquisition configuration and a first current is determined between said first electrode (31) and said second electrode (32) in said wet condition;
∘ a second relevant sub-phase in which said circuit (3) is in said third acquisition configuration and a third current is determined between said first electrode (31) and said second electrode (32) in said wet condition and an additional third current between said first electrode (31) and said fourth electrode (34) in said wet condition;
∘ a calculation sub-phase in which the concentration of a solute in said full-bodied fluid is determined as a function of said first current, said third current, said additional third current and said setting current.
